# EUROPEAN PATENT SPECIFICATION

(11) **EP 3 111 227 B1**
(45) Date of publication and mention of the grant of the patent: **21.08.2019**
(21) Application number: 15755681.2
(22) Date of filing: 26.02.2015
(51) Int. Cl.: G01N 33/68, G01N 33/569, C12Q 1/68, C12Q 1/04

(54) **METHOD FOR DIAGNOSING TUBERCULOSIS**
VERFAHREN ZUR DIAGNOSE VON TUBERKULOSE
MÉTHODE DE DIAGNOSTIC D'UNE TUBERCULOSE

(30) Priority: 26.02.2014 ZA 201401456
(43) Date of publication of application: 04.01.2017
(73) Proprietor: Stellenbosch University, 7600 Western Cape Province (ZA)
(72) Inventor: CHEGOU, Novel Njweipi, 7530 Cape Town (ZA); WALZL, Gerhard, 7550 Cape Town (ZA); MIHRET, Adane, Addis Ababa 1000 (ET)
(74) Representative: Haseltine Lake Kempner LLP
(86) International application number: PCT/IB2015/051435
(87) International publication number: WO 2015/128830

(56) References cited:
- WO-A2-2006/125973
- WO-A2-2013/175459
- NOVEL N CHEGOU ET AL: "Potential of novel Mycobacterium tuberculosis infection phase-dependent antigens in the diagnosis of TB disease in a high burden setting", BMC INFECTIOUS DISEASES, BIOMED CENTRAL, LONDON, GB, vol. 12, no. 1, 20 January 2012 (2012-01-20), page 10, XP021130535, ISSN: 1471-2334, DOI: 10.1186/1471-2334-12-10
- NOVEL N. CHEGOU ET AL: "Potential of Host Markers Produced by Infection Phase-Dependent Antigen-Stimulated Cells for the Diagnosis of Tuberculosis in a Highly Endemic Area", PLOS ONE, vol. 7, no. 6, 5 June 2012 (2012-06-05), page e38501, XP055073499, DOI: 10.1371/journal.pone.0038501
- MORTEN RUHWALD ET AL: "IP-10 release assays in the diagnosis of tuberculosis infection: current status and future directions", EXPERT REVIEW OF MOLECULAR DIAGNOSTICS, vol. 12, no. 2, 1 March 2012 (2012-03-01), pages 175-187, XP055073280, ISSN: 1473-7159, DOI: 10.1586/erm.11.97
- J. LIGHTER ET AL: "Chemokine IP-10: an adjunct marker for latent tuberculosis infection in children", INT J TUBERC LUNG DIS, vol. 13, no. 6, 1 June 2009 (2009-06-01), pages 731-736, XP055073278,
- CHEGOU, N.N. ET AL.: 'Utility of host markers detected in Quantiferon supematants for the diagnosis of tuberculosis in children in a high-burden setting.' PLOS ONE vol. 8, no. ISSUE, May 2013, page E64226, XP055220294
- MIHRET, A. ET AL.: 'Plasma cytokines and chemokines differentiate between active disease and nonactive tuberculosis infection.' JOURNAL OF INFECTION vol. 66, April 2013, pages 357 - 365, XP028991384
- WANG, C. ET AL.: 'Serum complement C4b, fibronectin, and prolidase are associated with the pathological changes of pulmonary tuberculosis.' BMC INFECTIOUS DISEASES vol. 14, no. 1-9, 2014, page 52, XP021175517
- DUFFY, D. ET AL.: 'The ABCs of viral hepatitis that define biomarker signatures of acute viral hepatitis.' HEPATOLOGY vol. 59, no. 4, April 2014, pages 1273 - 1282, XP055220297

## Description

### CROSS-REFERENCE TO RELATED APPLICATIONS

This application claims priority to South African provisional patent application number 2014/01456.

### FIELD OF THE INVENTION

This invention relates to a method for the diagnosis of tuberculosis in a subject.

### BACKGROUND TO THE INVENTION

With an estimated 9 million new cases and 1.5 million deaths each year, tuberculosis (TB) is a major global health problem and one of the developing world's biggest killers (85 percent of cases occur in Asia and Africa). In recent years there has been a dramatic resurgence of the disease in people also infected with HIV. In addition, drug-resistant strains of the disease are on the rise. Controlling the spread of TB is severely hampered by the fact that the disease is very difficult to both diagnose and treat, with new tools to tackle the disease severely lacking.

The most widely-used test for diagnosing active TB in developing countries relies on examining a patient's phlegm under a microscope (smear microscopy). This method, developed nearly 140 years ago, detects less than half of all active TB cases and in particular largely fails to detect the disease in children, people co-infected with HIV and those with drug-resistant forms of TB or extra-pulmonary disease. *Mycobacterium tuberculosis* (*M.tb*) culture facilities are also not widely available in resource-constrained settings and culture results may take up to 42 days to become available [5].

There has been a recent upswing of activity in the development of new TB diagnostic methods, but these still require laboratories, a steady power supply and skilled staff to deliver results which are mainly unavailable in remote and rural settings.

Advanced TB immunodiagnostic tests, such as the so called interferon gamma (IFN-γ) release assays (IGRAs), have proven to be useful in the diagnosis of *M.tb* infection [12-14] and are widely used in high income, low-burden settings. However, these assays do not discriminate between latent TB disease and active TB and are therefore not recommended in high burden settings [15]. Attempts have been made at identifying novel antigens other than those currently used in IGRAs (ESAT-6/CFP-10/TB7.7) [16-18], or novel host biomarkers produced in response to the antigens currently used in IGRAs [17,19-24] but these investigations are still on-going (reviewed in [24]).

Researchers have investigated differential cytokine secretion patterns after stimulation of blood cells with *M.tb* specific antigens [24]. These are mostly flow cytometry and ELISPOT based approaches and have demonstrated that enumeration of the frequencies and ratios of T cells producing IFN-γ and IL-2, for example, might have potential in the diagnosis of TB disease [25-28]. However, diagnostic approaches relying on the separation of isolated cells from whole blood are cumbersome and require laboratory capacity that is not usually available in resource constrained settings.

It has previously been shown that the measurement of host biomarkers other than IFN-γ in whole blood samples after stimulation with *M.tb* specific antigens has potential as a diagnostic modality for TB disease [19,21,22,24]. However, tests based on these host biomarkers (detected in Quantiferon supernatants) require overnight incubation of the Quantiferon tubes containing the samples and so results will only be available in 24 hours from the time of sample collection. This is also true in approaches based on the stimulation of whole blood from individuals suspected of TB disease with novel *Mtb* infection phase dependent antigens [16,20,30]. Diagnostic approaches based on the isolation of peripheral blood mononuclear cells from whole blood, stimulating the blood cells with *M.tb* specific antigens and evaluating the frequency of cells producing cytokines, especially IFN-γ, TNF-α and IL-2 by ELISPOT or flow cytometry [25-28] have shown potential in the diagnosis of TB disease. However, these techniques are cumbersome and more suitable for research laboratories and not routine hospital laboratories due to the high skill levels needed to isolate the cells and perform the ELISPOT and flow cytometry.

If assays based on these approaches continue to show promise, such tests will be based in highly centralized and specialized laboratories and not easily available in resource-limited settings, which often have the highest TB burdens. The consequent long time to availability of tests results will result in delays in diagnosis of the disease, delays in initiation of treatment of patients and consequently increased risk of transmission of the disease amongst contacts of the patients.

The Xpert MTB/RIF test (Cepheid Inc, CA, USA), an important advance in the field, yields results within 2 hours and is coupled with the detection of rifampicin resistance [6]. The test has a pooled sensitivity of 67-98% and specificity of about 98% in adults [7]. However, limitations such as high costs, the requirement for a stable electricity supply, centralised laboratory equipment, trained laboratory staff and short shelf-life of consumables amongst others [8], hamper the massive implementation of the test in resource-constrained and often high-burden settings.

There is thus a need for a simple, accurate and affordable TB test that can deliver results on the spot and is easy-to-use in resource-limited settings.

### SUMMARY OF THE INVENTION

The invention provides a method for the diagnosis of tuberculosis, the method comprising the step of testing an unstimulated blood sample from a subject suspected of having tuberculosis for at least 4 biomarkers, wherein the biomarkers are selected from the group consisting of complement factor H, of c-reactive protein (CRP), pre-albumin, interferon (IFN)-γ, apolipoprotein (APO)-A1 and interferon inducible protein (IP)-10.

Further embodiments of the invention are defined in and by the appended claims.

According to a first embodiment of the disclosure, there is provided a method for the diagnosis of tuberculosis, the method comprising the step of testing an unstimulated blood sample from a subject suspected of having tuberculosis for at least four, at least five or at least six biomarkers, wherein the biomarkers are selected from the group consisting of c-reactive protein (CRP), pre-albumin, interferon (IFN)-γ, complement factor H, apolipoprotein (APO)-A1, interferon inducible protein (IP)-10, complement C3, alpha-2-macroglobulin, APO-CIII, APO-E, interferon (IFN)-α2, tumour necrosis factor (TNF)-α, epidermal growth factor (EGF), macrophage inflammatory protein (MIP)-1β, soluble CD40 ligand (sCD40L), transforming growth factor (TGF)-α, vascular endothelial growth factor (VEGF), interleukin 1 receptor antagonist (IL-1ra), matrix metallo-proteinase (MMP)-2, matrix metallo-proteinase (MMP)-9, haptoglobulin, serum amyloid A (SAA), procalcitonin (PCT), ferritin, tissue plasminogen activator (TPA), fibrinogen and serum amyloid A (SAA).

Preferably, the biomarkers are selected from the group consisting of CRP, prealbumin, IFN-γ, complement factor H, apolipoprotein A1, IP-10, SAA, ferritin, fibrinogen, TGF-α, TPA, VEGF, PCT and TNF-α.

At least two, at least three, at least four or at least five of the biomarkers may be selected from CRP, prealbumin, IFN-γ, complement factor H, apolipoprotein A1 and/or IP-10 and the other biomarkers may be selected from SAA, Ferritin, Fibrinogen, TGF-α, TPA, VEGF, PCT and/or TNF-α.

More preferably, the biomarkers may be selected from the group consisting of CRP, prealbumin, IFN-γ, complement factor H, apolipoprotein A1 and IP-10.

Even more preferably, the method may comprise testing the sample for the following 6 biomarkers: CRP, prealbumin, IFN- γ, complement factor H, apolipoprotein A1 and IP-10.

A capture agent may be used to bind each of the biomarkers. The capture agents may be antibodies, affybodies, ankyrin repeat proteins, armadillo repeat proteins, nucleic acid aptamers, modified nucleic acid aptamers, peptides, modified peptides, carbohydrate ligands, synthetic ligands or synthetic polymers.

One or more indicators may be used to indicate when binding of each of the capture agents and biomarkers occurs. The indicator may be a calorimetric, electrochemical, chromogenic, optical, fluorescent or a radio-labeled indicator.

Levels of the biomarkers in the sample may be detected so as to determine whether the subject has TB or does not have TB. For example, a measured signal which equates to a level of biomarker in the sample which is higher than a threshhold level of the same biomarker may be an indicator of tuberculosis disease for the following biomarkers: IFN-α2, CRP, IFN-γ, IL-1ra, IP-10, TGF-α, TNF-α, VEGF, complement factor H, complement C3, PCT, SAA, ferritin, fibrinogen, TPA or MMP-9.

On the other hand, a measured signal which equates to a level of biomarker in the sample which is lower than a threshhold level of the same biomarker may be an indicator of tuberculosis disease for the following biomarkers: APO-A1, APO-CIII, pre-albumin or MMP-2.

The threshold level of the biomarkers may correspond to levels of the same biomarkers in subjects who do not have tuberculosis.

The blood sample may be whole blood, blood plasma or blood serum, which is either from whole blood which has been centrifuged or from blood which has been allowed to clot.

The tuberculosis may be pulmonary tuberculosis.

According to a second embodiment of the disclosure, there is provided a device for diagnosing tuberculosis according to the method described above, the device comprising:
a means for receiving a blood sample from a subject;
capture agents for binding at least four of the above listed biomarkers if the biomarkers are present in the sample; and
at least one indicator which indicates when the capture agents bind to the biomarkers.

The device may be a device according to any one of paragraphs 20 to 22 of the numbered paragraphs immediately before the claims, which includes measuring means for measuring the levels of the detected biomarkers.

A device may be a hand-held point-of-care device, and may further include amplifying means for increasing the sensitivity of the detection of the biomarkers, such as up-converting phosphor technology.

The device may be a point-of-care device, such as a hand held device, and more particularly a lateral flow device.

According to a third embodiment of the disclosure, there is provided a kit for diagnosing tuberculosis in a blood sample from a subject according to the method described above, the kit comprising:
a means of receiving a sample from a patient;
at least one capture agent for binding at least four of the above listed biomarkers; and at least one indicator which indicates when the capture agents bind to the biomarkers.

The kit may further include a device as described above.

### BRIEF DESCRIPTION OF THE FIGURES

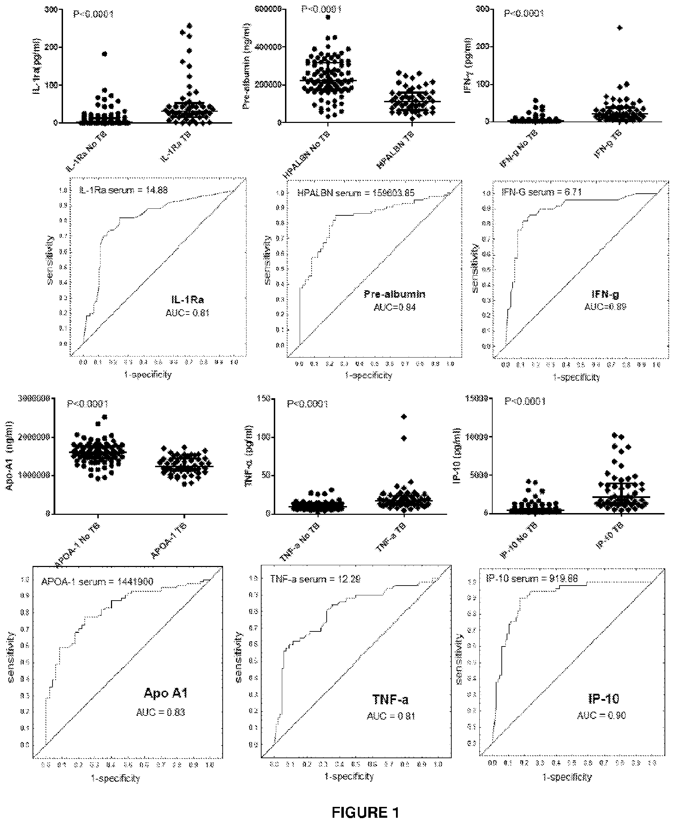

- Figure 1: shows levels of host biomarkers detected in serum samples from pulmonary TB cases and individuals without TB disease and receiver operator characteristics (ROC) plots showing accuracies of these biomarkers in the diagnosis of TB disease, regardless of HIV infection, in a pilot study. Error bars in the scatter-dot plots indicate the median and Inter-quartile ranges. Representative plots for analytes with area under the ROC curve (AUC) ≥ 0.70;

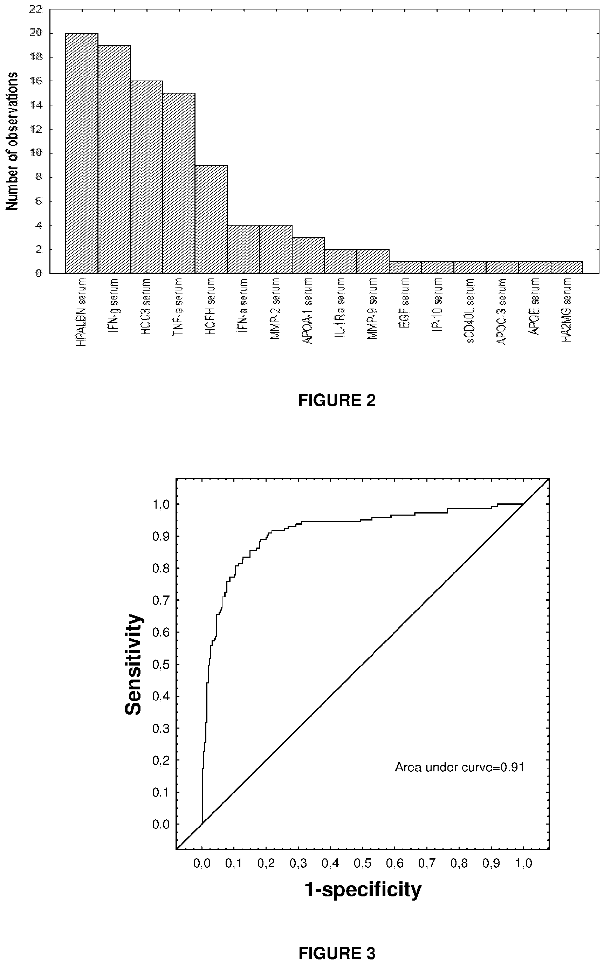

- Figure 2: shows frequency of analytes in the top 20 general discriminant analysis (GDA) predictive models that most accurately classified study participants as TB disease or no TB disease in the pilot study (n=148 study participants). The columns represent the number of times each analyte was included in the top 20 discriminatory models. Study participants were not stratified according to HIV infection status;
- Figure 3: is the receiver operator characteristics (ROC) curve showing the accuracy of a 6-marker biosignature in the diagnosis of active TB disease, as obtained in a validation study (n=703 study participants);

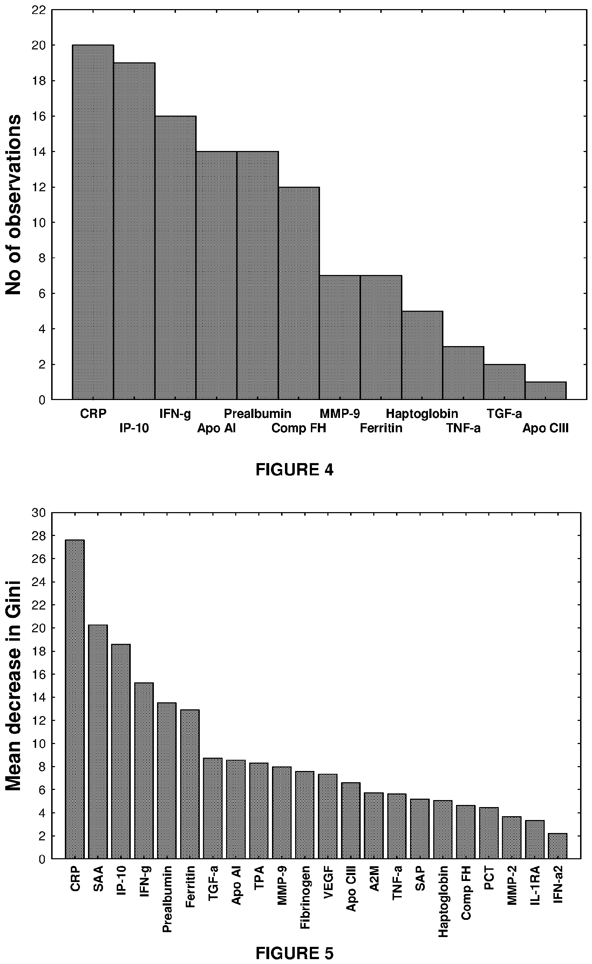

- Figure 4: shows frequency of analytes in the top 20 general discriminant analysis (GDA) predictive models that most accurately classified study participants as TB disease or no TB disease in the validation study (n=703 study participants). The columns represent the number of times each analyte was included in the top 20 discriminatory models. Study participants were not stratified according to HIV infection status or ethnicity; and
- Figure 5: shows the importance of analytes in multi-marker predictive models for the classification of study participants as TB disease or no TB disease in the validation study (n=703 study participants), as revealed by the random forest analysis technique. The columns represent the importance of the analytes in the multi-marker models.

### DETAILED DESCRIPTION OF THE INVENTION

A method for diagnosing tuberculosis (TB) and a device for performing the method are described herein. The method comprises the step of detecting a panel of at least four biomarkers in an unstimulated blood sample from a subject, the biomarkers being selected from the group consisting of complement C3, complement factor H, alpha-2-macroglobulin, apolipoprotein (APO)-A1, apolipoprotein APO-CIII, apolipoprotein APO-E, pre-albumin interferon (IFN)-γ, interferon (IFN)-α2, interferon inducible protein (IP)-10, tumour necrosis factor (TNF)-α, epidermal growth factor (EGF), macrophage inflammatory protein (MIP)-1β, soluble CD40 ligand (sCD40L), transforming growth factor (TGF)-α, vascular endothelial growth factor (VEGF), interleukin 1 receptor antagonist (IL-1ra), matrix metallo-proteinase (MMP)-2, matrix metallo-proteinase (MMP)-9, haptoglobulin, c-reactive protein (CRP), serum amyloid A (SAA), procalcitonin (PCT), ferritin, tissue plasminogen activator (TPA), fibrinogen and serum amyloid A (SAA). This panel can be referred to as a biosignature panel.

The panel may include more than four biomarkers from the list described above so as to provide a more comprehensive test and to reduce false negative results, such as including five, six, seven or even more of the biomarkers listed above.

As used herein, the terms "comprises", "comprising", "includes", "including", "contains", "containing", and any variations thereof, are intended to cover a non-exclusive inclusion and do not exclude other elements not specifically mentioned.

The terms "marker" and "biomarker" are used interchangeably to refer to a target molecule that indicates or is a sign of a normal or abnormal process in an individual or of a disease or other condition in an individual. More specifically, a "marker" or "biomarker" is an anatomic, physiologic, biochemical, or molecular parameter associated with the presence of a specific physiological state or process, whether normal or abnormal. Biomarkers can include small molecules, peptides, proteins, and nucleic acids.

Biomarkers are generally described as being over-expressed, under-expressed or differentially expressed as compared to an expression level or value of the biomarker that indicates or is a sign of a normal process or an absence of a disease or other condition in an individual. Thus, "over-expression", "under-expression" and "differential expression" of a biomarker can also be referred to as a variation from a "normal" or "control" expression level of the biomarker.

In one embodiment, the biomarkers can be selected from the group consisting of CRP, prealbumin, IFN-γ, complement factor H, apolipoprotein A1, IP-10, SAA, ferritin, fibrinogen, TGF-α, TPA, VEGF, PCT and TNF-α.

In another embodiment, at least two, at least three, at least four or at least five of the biomarkers in the biomarker panel can be selected from any of CRP, prealbumin, IFN-γ, complement factor H, apolipoprotein A1 and/or IP-10, and the other biomarkers in the panel can be selected from any of SAA, Ferritin, Fibrinogen, TGF-α, TPA, VEGF, PCT and/or TNF-α.

In yet another embodiment, the biomarkers in the panel can be selected from any of CRP, prealbumin, IFN-γ, complement factor H, apolipoprotein A1 and/or IP-10.

In yet a further embodiment, the biomarker panel includes the following six biomarkers: CRP, prealbumin, IFN-γ, complement factor H, apolipoprotein A1 and IP-10.

In yet a further embodiment of the disclosure, one or more of the biomarkers in the panel of CRP, prealbumin, IFN-γ, complement factor H, apolipoprotein A1 and IP-10 may be replaced with any of the following biomarkers: SAA, Ferritin, Fibrinogen, TGF-α, TPA, VEGF, PCT and/or TNF-α.

Capture agents are used to bind specifically to each of the biomarkers, and an indicator will indicate when binding of the capture agents and each of the biomarkers occurs. The capture agent is typically an antibody, but examples of other possible capture agents include affybodies, ankyrin repeat proteins, armadillo repeat proteins, nucleic acid aptamers, modified nucleic acid aptamers, peptides, modified peptides, carbohydrate ligands, synthetic ligands, and synthetic polymers. The indicator can be a calorimetric, electrochemical, chromogenic, optical, fluorescent or a radio-labeled indicator.

Levels of the biomarkers in the sample can be detected so as to determine whether the subject has TB or does not have TB. Cut-off or threshold values can be determined based on levels of biomarkers which are typically found in patients with active TB or in subjects without TB, and the levels of the biomarkers detected in the sample can be compared to the cut-off levels when making the determination of whether or not the subject has TB. In other words, the method will detect whether the biomarkers in the panels are under- or over-expressed relative to a subject who does not have TB. Generally, all of the biomarkers in the panel must either be over-expressed or under-expressed in the sample (depending on what is shown in Table 2) in order for the method to provide a positive TB diagnosis.

The blood sample can be whole blood, plasma or serum, depending on how the method will be performed. For example, in one embodiment, the blood sample will be allowed to clot and the serum will then be analysed to detect the biomarkers. In an alternative embodiment, the blood sample will not be allowed to clot (e.g. an anticoagulant can be added to the sample) and instead the blood cells and/or clotting factors will be separated from the sample, e.g. by centrifuge. The plasma can then be analysed to detect the biomarkers. In another embodiment, a whole blood sample, such as from a finger-prick, can be analysed directly.

The TB is typically pulmonary TB.

The method of the invention can be performed using a diagnostic device which detects and indicates the presence of the biomarkers in the sample. The device has a means for receiving the sample from the subject, such as a loading or receiving area onto or into which the sample is placed. The capture agents and indicators are present in the device, and once the sample has been loaded onto or received into the device, the sample is brought into contact with the capture agents, which are allowed to bind to the biomarkers if present. The indicator will signify that binding has occurred. The device is typically a point-of-care device, such as a lateral flow device. A hand-held device is in the process of being developed which is capable of making a TB diagnosis based on a lateral flow assay using up-converting phosphor technology [31-34].

In one embodiment, the device may be a dip-stick which can be dipped into the blood sample or onto which the blood sample can be placed, similar to many home pregnancy test kits. The capture agents are included on the dipstick, and generate a signal when they bind to the biomarkers in the panel, together with a control signal.

A kit can also be provided for performing the invention, and can include one or more of the following:
a means for collecting the sample from the patient (such as a needle);
receptacles for receiving the blood sample from the subject;
capture agents for specifically binding to the biomarkers;
indicators for indicating when the capture agents have bound to biomarkers;
capture agents for binding at least two of the biomarkers described above;
a device as described above; and/or
instructions for performing the method of diagnosis described above.

Unlike other commercially available methods, the present method can distinguish between active and latent tuberculosis, which is important for determining treatment of the subject. Active tuberculosis is the disease state where the biological fluid or tissue is smear microscopy- or culture positive for *M*. *tuberculosis,* or where *M*. *tuberculosis* is detectable by a nucleic acid amplification test, in a subject with clinical and radiological features of active TB disease; constitutional symptoms are often present. Latent tuberculosis infection is where potentially viable *M*. *tuberculosis* is present in human tissues but the individual is asymptomatic, and without clinico-radiological features of active disease.

The invention will now be described in more detail by way of the following non limiting examples.

### Examples

### Materials and methods

The study was conducted in two stages. In the pilot/discovery phase of the project, 148 individuals that presented with symptoms requiring investigation for TB disease at peripheral health centres in Cape Town, South Africa and Addis Ababa, Ethiopia were recruited. These individuals were enrolled as part of the ongoing EDCTP-funded African European Tuberculosis Consortium (AE-TBC) study (www.ae-tbc.eu), which aims to identify biomarkers for the diagnosis of tuberculosis disease at seven different field sites in six African countries. In the second (validation) phase of the project, 718 study participants from five of the sites participating in the project (Stellenbosch University in South Africa, MRC Unit in the Gambia, Makerere University in Uganda, Karonga Prevention Study (KPS) in Malawi and the University of Namibia (UNAM)) were evaluated.

All the participants included in the study presented at the respective rural health care facilities with symptoms suggestive of pulmonary TB disease. These included persistent coughing lasting ≥2 weeks and at least one of either fever, malaise, recent weight loss, night sweats, knowledge of close contact with a TB patient, haemoptysis, chest pain or loss of appetite. All participants enrolled into the study were 18 years or older and willing to give written informed consent for participation in the study, including for HIV testing. Patients were excluded if they had not been residing in the study community for more than 3 months, were severely anaemic (HB<10g/l), on anti-TB treatment, had received anti-TB treatment in the previous 90 days or if they were on quinolone or aminoglycoside antibiotics in the past 60 days. At enrolment, a case report form was completed for each participant before blood and other samples required for the main study, were collected. The study was approved by the Health Research Ethics Committees of the different institutions participating in the project.

### Sample collection and diagnostic tests

Harmonized protocols were used for collection and processing of samples across all the sites involved in the study. Briefly, blood samples were collected into 4ml plain BD vacutainer® tubes (BD Biosciences) and transported at ambient conditions to the laboratory. The tubes were then centrifuged at 2500 rpm for 10 minutes after which serum was harvested, aliquoted and frozen (-80°C) until use. Sputum samples were collected from all participants and cultured using the MGIT method (BD Biosciences). PCR experiments were performed on all positive MGIT samples to confirm the isolation of *M.tb* complex organisms. All the individuals classified as TB cases had positive *M.tb* complex speciated sputum cultures and/or other clinical features in keeping with TB, including typical chest X-rays. The individuals in the "No TB" group had negative sputum smears and cultures and had no other signs suggestive of TB including negative chest X-rays. None of the non-TB cases were treated for TB by the respective national TB control programs.

### Luminex multiplex immunoassay

The levels of biomarkers previously tested in stimulated supernatants, including interferon (IFN)-γ, IFN-α2, interferon inducible protein (IP)-10, tumour necrosis factor (TNF)-α, epidermal growth factor (EGF), macrophage inflammatory protein (MIP)-1β, soluble CD40 ligand (sCD40L), transforming growth factor (TGF)-a, vascular endothelial growth factor (VEGF), interleukin 1 receptor antagonist (IL-1ra), matrix metallo-proteinase (MMP)-2, and MMP-9 and new host biomarkers including complement C3, complement factor H, alpha-2-macroglobulin, apolipoprotein (APO)-A1, APO-CIII, APO-E and pre-albumin, were evaluated in serum samples from all study participants in phase 1 of the study. In the second phase, additional biomarkers, i.e. haptoglobulin, c-reactive protein (CRP), serum amyloid A (SAA), procalcitonin (PCT), ferritin, tissue plasminogen activator (TPA), fibrinogen and serum amyloid A (SAA), were evaluated on serum samples on all study participants included in the validation study. This was done using Milliplex kits (Merck Millipore, St. Charles, Missouri, USA) or Bio Plex kits (Bio Rad laboratories, Hercules, CA, USA), on the Bio-Plex™ platform (Bio-Rad Laboratories, Hercules, CA, USA). Samples for MMP-2 and MMP-9 were pre-diluted 1:100 following optimization experiments, samples for ferritin, fibrinogen, SAA, PCT, TPA were diluted 1:100 and those for CRP, A2M, haptoglobulin and SAP were diluted 1:10000 as recommended by the manufacturer (Bio Rad). The laboratory staff performing the experiments were blinded to the clinical groups of study participants. Assays were performed according to the instructions of the manufacturers (Merck Millipore and Bio Rad) and the levels of all analytes in the quality control reagents were within the manufacturer's recommended ranges. The Bio-Plex Manager™ Software 6.1 was used for bead acquisition and analysis.

### Statistical analysis

Differences in analyte levels between the participants with TB disease and those without active TB were evaluated by the Mann-Whitney U test for non-parametric data analysis. The diagnostic accuracy of individual biomarkers was investigated by receiver operator characteristics (ROC) curve analysis. Optimal cut-off values and associated sensitivity and specificity were selected based on the Youdens Index approach. The predictive abilities of combinations of analytes were estimated by performing best subsets general discriminant analysis (GDA), with leave-one-out cross validation in the pilot study, or the training and test set approach in the validation study. In the validation study, a second multi-marker analysis procedure (random forest) was employed investigating the accuracy of the serum biosignatures. Differences between groups were considered significant if p-values were <0.05. Data were analyzed using GraphPad prism, version 5.00 (GraphPad Software, San Diego, California, USA) and Statistica (Statsoft, Ohio, USA).

### Results

A total of 148 participants were included in the pilot study. Of these participants, 72 (49%) were males and 76 (51%) were females. The mean age of all pilot study participants was 34.7±12.3 and 22 (15%) of the study participants were HIV infected. A total of 92 (63%) of all these study participants were Quantiferon positive, using the manufacturer's recommended cut-off value (≥0.35IU/ml). Fifty-one (34.5%) of the study participants were confirmed with TB disease, 88 (59.5%) with no TB, while 9 (6.0%) had an uncertain diagnosis (could not be classified as TB or no TB disease using the current standard laboratory and clinical methods). The 9 individuals with an uncertain diagnosis were excluded from further analysis.

The validation study included a total of 718 study participants. Samples from 15 individuals were excluded from final analysis for the following reasons: 10 of the study participants were pregnant or had conflicting data between the clinical and study research teams, possibly as a result of human laboratory errors, and 5 of the study participants were questionable TB cases (could not be classified as TB or no TB with certainty using the currently available diagnostic tools). Of the 703 eligible study participants,358 (51%) were of the male gender and 173 (25%) were HIV infected. The mean age of all study participants was 36±11.8, 374 (55%) were QFT positive and 28 (4%) were QFT indeterminate, using the manufacturer's cut-off value (≥0.35IU/ml).

### Performance of individual biomarkers in the diagnosis of TB disease in the pilot phase of the study (n = 148 study participants)

The median levels of IFN-α2, IFN-γ, IL-1ra, IP-10, TGF-α, TNF-α, VEGF, complement factor H, complement C3 and MMP-9 were significantly higher in the TB cases, while the levels of APO-A1, APO-CIII, pre-albumin and MMP-2 were higher in the individuals without TB disease. This was irrespective of the HIV infection status of study participants. The most accurate individual analytes, as determined by AUC >0.80 included IFN-γ, IL-1ra, IP-10, TNF-α, APO-A1 and pre-albumin. The median levels of all the biomarkers evaluated in the study and measures of diagnostic accuracy are shown in Table 1, while representative ROC plots are shown in Figure 1.

### Performance of individual biomarkers in the diagnosis of TB disease in the validation phase of the study (n = 703 study participants)

The median levels of IL-1ra, TGF-α, IP-10, TNF-α, IFN-α2, IFN-γ, VEGF, MMP-9, complement factor H, haptoglobulin, CRP, SAP, PCT, ferritin, TPA, fibrinogen and SAA were significantly higher in the TB cases, whereas the levels of pre-albumin and the two apolipoproteins (APO-A1 and APO-CIII) were significantly higher in the non-TB cases. The differences in the concentrations of MMP-2 (p=0.09) and A2M (p=0.14); both higher in the non-TB cases, were not significant. (Table 2).

**Table 1: Pilot study: median levels of analytes detected in serum samples from individuals with pulmonary TB or no TB disease (n=148), inter-quartile ranges (in parenthesis), and accuracies in the diagnosis of TB disease.**

| **Host Marker** | **No TB (n=88)** | **TB (n=50)** | **P-value** | **AUC** | **Cut-off** | **Sensitivity (%)** | **Specificity (%)** |
|---|---|---|---|---|---|---|---|
| EGF | 94 (57-183) | 112 (63-166) | 0.479 | 0.54 | 96 | 66 | 51 |
| IFN-α2 | 15 (9-25) | 30 (17-46) | <0.0001 | 0.74 | 28 | 60 | 84 |
| **IFN-γ** | **3 (2-5)** | **21 (14-38)** | **<0.0001** | **0.89** | **7** | **86** | **84** |
| **IL-1ra** | **3 (0-13)** | **31(15-52)** | **<0.0001** | **0.81** | **15** | **82** | **76** |
| **IP-10** | **434 (337-624)** | **2072 (1256-3789)** | **<0.0001** | **0.90** | **920** | **90** | **83** |
| MIP-1β | 39 (29-58) | 40(25-61) | 0.706 | 0.52 | 42 | 48 | 56 |
| sCD40L | 53474 (43228-188820) | 65089 (45638-254369) | 0.110 | 0.58 | 55041 | 66 | 52 |
| TGF-α | 6 (3-11) | 11 (7-18) | <0.001 | 0.71 | 9 | 70 | 64 |
| **TNF-α** | **10(8-13** | **17(13-23)** | **<0.0001** | **0.81** | **12** | **82** | **67** |
| VEGF | 156 (99-256) | 287 (184-468) | <0.0001 | 0.72 | 175 | 86 | 56 |
| **APO-A1** | **1599200 (1451850-1741800)** | **1239850 (1123500-1438600)** | **<0.0001** | **0.83** | **1441900** | **77** | **76** |
| APO-CIII | 191503 (150363-257175) | 148359 (113696-187681) | <0.001 | 0.70 | 178825 | 60 | 74 |
| APO-E | 81649 (62736-108447) | 72292 (58174-103665) | 0.256 | 0.56 | 74331 | 64 | 54 |
| **Pre-albumin** | **222732 (177274-316267)** | **116014 (76521-159273)** | **<0.0001** | **0.84** | **159604** | **85** | **76** |
| CFH | 313921 (267466-375045) | 358055 (306217-442913) | 0.004 | 0.65 | 322902 | 68 | 56 |
| CC3 | 7424 (3820-10120) | 10287 (6536-17060) | 0.0005 | 0.68 | 9302 | 62 | 68 |
| A2MG | 2071850 (1557750-2453050) | 1891850 (1668300-2490800) | 0.504 | 0.53 | 1912500 | 58 | 58 |
| MMP-2 | 307755(240689 -315081) | 240688 (197351-307755) | 0.001 | 0.66 | 279484 | 59 | 68 |
| MMP-9 | 344088(217046 -592648) | 658950 (377276-993065) | <0.001 | 0.72 | 47621 | 66 | 67 |

| | | | | | | | |
|---|---|---|---|---|---|---|---|
| ; CFH= complement factor H, CC3= complement C3, A2MG=alpha 2 macroglobulin; AUC = Area under the ROC curve Both HIV infected and uninfected individuals were included in the analysis. The values shown for EGF, IFN-α2, IFN-γ, IL-1ra, IP-10, MIP-1β, sCD40L, TGF-α, TNF-α and VEGF are in pg/ml. All other analyte levels are in ng/ml. | | | | | | | |

**Table 2: Validation study: median levels of analytes detected in serum samples from individuals with pulmonary TB or no TB disease (n=703), inter-quartile ranges (in parenthesis), and accuracies in the diagnosis of TB disease.**

| **Host marker** | **Median in no TB (n=487)** | **Median in TB (n=215)** | **P-value** | **AUC** | **Sensitivity** (%) | **Specificity** (%) |
|---|---|---|---|---|---|---|
| IL-1ra | 8 (0-40) | 35 (0-77) | **<0,000001** | 0,59 | 52 | 70 |
| TGF-α | 3 (1-6) | 7 (3-13) | **<0,000001** | 0,76 | 66 | 76 |
| IP-10 | 368 (209-652) | 1712 (808-3558) | **<0,000001** | 0,83 | 81 | 78 |
| TNF-α | 7(3-12) | 14 (8-27) | **<0,000001** | 0,70 | 67 | 66 |
| IFN-α2 | 0 (0-6) | 7 (0-19) | **<0,000001** | 0,66 | 59 | 70 |
| IFN-γ | 1 (0-3) | 9(3-21) | **<0,000001** | 0,79 | 77 | 76 |
| VEGF | 158 (19-286) | 341 (144-624) | **<0,000001** | 0,72 | 60 | 76 |
| MMP-2 | 175792 (28693-474927) | 92881 (22348-312697) | **0,0916** | 0,52 | 67 | 42 |
| MMP-9 | 401540 (155072-756297) | 651549 (43831-1299700) | **0,00039** | 0,62 1299700) | 56 | 72 |
| Apo Al | 2593900 (2101500-3847700) | 1999800 (1493900-2604300) | **<0,000001** | 0,72 | 76 | 59 |
| Apo CIII | 261321 (178708-418395) | 180967 (115790-297779) | **<0,000001** | 0,65 | 75 | 51 |
| Prealb | 411528 (261059-591773) | 184107 (107526-291488) | 184107 **<0,000001** | 0,79 | 72 | 76 |
| CFH | 663345 (515681-929872) | 760622 (599474-1008200) | **0,0013** | 0,58 | 53 | 63 |
| A2M | 1770000 (712956-3273400) | 1380700 (501530-3284700) | 0,1412 | 0,50 | 47 | 59 |
| Hapto | 955718 (287186-26796000) | 2774400 (443581-60000000) | **0,000001** | 0,62 | 50 | 73 |
| CRP | 1731 (321-9686) | 59195 (14047-136520) | **<0,000001** | 0,86 | 82 | 77 |
| SAP | 46609 (23028-81115) | 63664 (20776-129181) | **0,0011** | 0,62 | 58 | 67 |
| PCT | 4259 (2474-6776) | 6807 (4399-10000) | **0,000001** | 0,70 | 71 | 65 |
| Ferritin | 33894 (13921-83571) | 158610 (61712-365165) | **0,000001** | 0,80 | 81 | 67 |
| TPA | 1638 (931-2604) | 2977 (1949-4317) | **0,000001** | 0,74 | 70 | 71 |
| Fibrin | 2466 (1804-4182) | 3987 (2991-6555) | **0,000001** | 0,76 | 78 | 65 |
| SAA | 771 (279-3985) | 6778 (4265-9689) | **0,000001** | 0,85 | 83 | 77 |

| | | | | | | |
|---|---|---|---|---|---|---|
| CFH= complement factor H, A2M=alpha 2 macroglobulin; CRP= C-reactive protein; SAP= serum amyloid P; SAA= serum amyloid A; PCT=procalcitonin; TPA= tissue plasminogen activator; prealb= pre-albumin, hapto=haptoglobulin; Fibrin=fibrinogen; AUC = Area under the ROC curve. Both HIV infected and uninfected individuals were included in the analysis. The values shown for IFN-α2, IFN-γ, IL-1 ra, IP-10, TGF-α, TNF-α, VEGF, ferritin, PCT and TPA are in pg/ml. All other analyte levels are in ng/ml. AUCs were generated from the training data prior to the general discriminant analysis. | | | | | | |

### Utility of serum multi-marker models in the diagnosis of TB disease

When the data obtained for all pilot study participants (regardless of HIV infection status) was fitted into general discriminant analysis models, optimal prediction of TB disease was achieved when biomarkers were used in combinations of five. A model comprising IFN-γ, TNF-α, pre-albumin, complement C3 and MMP-2 ascertained TB disease with a sensitivity of 86% (43/51) and specificity of 90.9% (80/88) in the training sample set, and sensitivity of 86% (43/51) and specificity of 89.8% (79/88) after leave-one-out cross validation. After bootstrap validation, the prediction accuracy of the model was 84% (confidence interval, 74-86%) for the TB cases and 93% (confidence interval, 89-95%) for the individuals without active TB disease. The analytes that occurred the most in the top 20 GDA models that most accurately classified study participants as active TB disease or no active TB disease included pre-albumin, IFN-γ, complement C3 and TNF-α (Figure 2). The 148 individuals included in this phase of the project were recruited from South Africa and Addis Ababa in Ethiopia.

The most promising markers identified in the pilot phase of the project and new host markers were evaluated in a larger study (n=703) in the validation phase, which was conducted in five African countries (South Africa, The Gambia, Uganda, Malawi and Namibia). When the data obtained on all 703 study participants was fitted into general discriminant analysis models, as was done in the pilot study, optimal prediction of TB disease was achieved when markers were used in combinations of six. The most accurate biosignature for the diagnosis of TB disease, regardless of HIV infection status or ethnicity was a combination of CRP, pre-albumin, IFN-γ, complement factor H, apolipoprotein A1 and IP-10. The AUC for this 6-marker biosignature (obtained from the training data set) was 0.91. The sensitivity of the biosignature in the training sample set (n=480) was 86%, with a specificity of 85%. The accuracy of the biosignature in the test dataset (n=207) was 89%, confidence interval: 78 to 95% 76% and the specificity was 76% (confidence interval: 68-83%) with a positive predictive value of 61.1% and a negative predictive value of 94.0%. The ROC curve for this biosignature is shown in Figure 3, and the number of times each host marker appeared in the top 20 GDA biomarker combinations is shown in Figure 4.

When the data generated during the validation phase of the study was analyzed using another multi-marker analysis procedure (random forest), a similar accuracy of the biosignature was obtained, thereby indicating the robustness of the biosignature. The sensitivity of the 6-marker biosignature in the training sample set was 84% and the specificity was 86%. In the test sample set, the sensitivity was 89% and the specificity 73%. The most important analytes in the discriminatory models for TB and no TB disease, regardless of HIV infection status or ethnicity, as revealed by the random forest analysis technique, were similar to what was obtained with the general discriminant analysis (Figure 5).

### Discussion

The applicant has shown that TB disease can be diagnosed by simply collecting blood from an individual suspected of having TB, allowing the blood to clot for a few minutes and then measuring the levels of a combination of host biomarkers in the serum. The advantage of this diagnostic approach is that the sample type needed for the study (serum, collected into a dry tube, without the requirement of any additives), is easy to obtain, implying that the test would be suitable in all patient types including children, and the biomarkers could be easily incorporated into a lateral flow device. Hence the test could be easily performed even in the most remote settings. The results, using multiple analysis strategies, indicate that a combination of 6 biomarkers is able to diagnose TB disease with a sensitivity of up to 89% (confidence interval, 78-95%) and specificity of 76% (confidence interval, 68-83%). The results of these investigations, using 851 samples from TB suspects recruited from six different sites in Africa, where individuals have diverse genetic backgrounds coupled with different circulating *M.tb* strains, indicate that a diagnostic test based on these biomarkers has the potential to contribute significantly to the diagnosis of TB disease and could be used, at least, as a screening test for TB in remote settings, before further investigations, if necessary.

In this study, novel biomarkers (complement C3, complement factor H, alpha-2-macroglobulin, apolipoprotein-A1, apolipoprotein-CIII, apolipoprotein-E, pre-albumin, ferritin, fibrinogen, tissue plasminogen activator, and procalcitonin) which, to the best of the applicant's knowledge, have never been investigated in serum samples for TB diagnostic purposes, were shown to be candidate diagnostic biomarkers for TB disease. These investigations were performed on serum samples from "TB suspects" and not a case-control study which usually tends to over-estimate the diagnostic accuracy. Although the novel biomarkers showed potential individually in the diagnosis of TB disease, observations indicate that the highest diagnostic accuracy is achieved if the biomarkers are used in combination with other established biomarkers, such as IFN-γ, IP-10, TNF-α, CRP, SAA and others.

It is envisaged that an assay according to the invention, employing host markers that could be directly measured in serum, will be faster and easier to perform than assays requiring overnight culture after stimulation of blood or blood cells with *M.tb* antigens, and would achieve a greater impact on patients since the time taken to diagnosis will be significantly reduced.

### References:

1. World Health Organisation. Global Tuberculosis Report 2012. 2012. Ref Type: Report
2. Murray CJ, DeJonghe E, Chum HJ, Nyangulu DS, Salomao A, Styblo K: Cost effectiveness of chemotherapy for pulmonary tuberculosis in three sub-Saharan African countries. Lancet 1991, 338: 1305-1308.
3. Apers L, Mutsvangwa J, Magwenzi J, Chigara N, Butterworth A, Mason P et al.: A comparison of direct microscopy, the concentration method and the Mycobacteria Growth Indicator Tube for the examination of sputum for acid-fast bacilli. Int J Tuberc Lung Dis 2003, 7: 376-381.
4. Chegou NN, Hoek KG, Kriel M, Warren RM, Victor TC, Walzl G: Tuberculosis assays: past, present and future. Expert Rev Anti Infect Ther 2011, 9: 457-469.
5. Hanna BA, Ebrahimzadeh A, Elliott LB, Morgan MA, Novak SM, Rusch-Gerdes S et al.: Multicenter evaluation of the BACTEC MGIT 960 system for recovery of mycobacteria. J Clin Microbiol 1999, 37: 748-752.
6. Boehme CC, Nabeta P, Hillemann D, Nicol MP, Shenai S, Krapp F et al.: Rapid molecular detection of tuberculosis and rifampin resistance. N Engl J Med 2010, 363: 1005-1015.
7. Steingart KR, Sohn H, Schiller I, Kloda LA, Boehme CC, Pai M et al.: Xpert(R) MTB/RIF assay for pulmonary tuberculosis and rifampicin resistance in adults. Cochrane Database Syst Rev 2013, 1: CD009593.
8. Trebucq A, Enarson DA, Chiang CY, Van DA, Harries AD, Boillot F et al.: Xpert((R)) MTB/RIF for national tuberculosis programmes in low-income countries: when, where and how? Int J Tuberc Lung Dis 2011, 15: 1567-1572.
9. Marais BJ, Pai M: New approaches and emerging technologies in the diagnosis of childhood tuberculosis. Paediatr Respir Rev 2007, 8: 124-133.
10. Chegou NN, Walzl G, Bolliger CT, Diacon AH, van den Heuvel MM: Evaluation of adapted whole-blood interferon-gamma release assays for the diagnosis of pleural tuberculosis. Respiration 2008, 76: 131-138.
11. Munk ME, Arend SM, Brock I, Ottenhoff TH, Andersen P: Use of ESAT-6 and CFP-10 antigens for diagnosis of extrapulmonary tuberculosis. J Infect Dis 2001, 183: 175-176.
12. Hesseling AC, Mandalakas AM, Kirchner HL, Chegou NN, Marais BJ, Stanley K et al.: Highly discordant T cell responses in individuals with recent exposure to household tuberculosis. Thorax 2009, 64: 840-846.
13. Mandalakas AM, Hesseling AC, Chegou NN, Kirchner HL, Zhu X, Marais BJ et al.: High level of discordant IGRA results in HIV-infected adults and children. Int J Tuberc Lung Dis 2008, 12: 417-423.
14. Mandalakas AM, Detjen AK, Hesseling AC, Benedetti A, Menzies D: Interferon-gamma release assays and childhood tuberculosis: systematic review and meta-analysis. Int J Tuberc Lung Dis 2011, 15: 1018-1032.
15. World Health Organization and the International Union Against Tuberculosis and Lung Disease. Guidance for national tuberculosis and HIV programmes on the management of tuberculosis in HIV-infected children: Recommendations for a public health approach. 2010. Ref Type: Report
16. Chegou NN, Black GF, Loxton AG, Stanley K, Essone PN, Klein MR et al.: Potential of novel Mycobacterium tuberculosis infection phase-dependent antigens in the diagnosis of TB disease in a high burden setting. BMC Infect Dis 2012, 12: 10.
17. Goletti D, Carrara S, Mayanja-Kizza H, Baseke J, Mugerwa MA, Girardi E et al.: Response to M. tuberculosis selected RD1 peptides in Ugandan HIV-infected patients with smear positive pulmonary tuberculosis: a pilot study. BMC Infect Dis 2008, 8:11.
18. Sartain MJ, Slayden RA, Singh KK, Laal S, Belisle JT: Disease state differentiation and identification of tuberculosis biomarkers via native antigen array profiling. Mol Cell Proteomics 2006, 5: 2102-2113.
19. Chegou NN, Black GF, Kidd M, van Helden PD, Walzl G: Host markers in Quantiferon supernatants differentiate active TB from latent TB infection : preliminary report. BMC Pulm Med 2009, 9: 21.
20. Chegou NN, Essone PN, Loxton AG, Stanley K, Black GF, van der Spuy GD et al.: Potential of host markers produced by infection phase-dependent antigen-stimulated cells for the diagnosis of tuberculosis in a highly endemic area. PLoS ONE 2012, 7: e38501.
21. Chegou NN, Detjen AK, Thiart L, Walters E, Mandalakas AM, Hesseling AC et al.: Utility of host markers detected in quantiferon supernatants for the diagnosis of tuberculosis in children in a high-burden setting. PLoS ONE 2013, 8: e64226.
22. Frahm M, Goswami ND, Owzar K, Hecker E, Mosher A, Cadogan E et al.: Discriminating between latent and active tuberculosis with multiple biomarker responses. Tuberculosis (Edinb) 2011, 91: 250-256.
23. Kellar KL, Gehrke J, Weis SE, Mahmutovic-Mayhew A, Davila B, Zajdowicz MJ et al.: Multiple cytokines are released when blood from patients with tuberculosis is stimulated with Mycobacterium tuberculosis antigens. PLoS ONE 2011, 6: e26545.
24. Novel N.Chegou, Jan Heyckendorf, Gerhard Walzl, Christoph Lange, Morten Ruhwald. Beyond the Interferon-gamma horizon: Biomarkers for immunodiagnosis of infection with M. tuberculosis. Eur Respir J . 2013. Ref Type: In Press
25. Biselli R, Mariotti S, Sargentini V, Sauzullo I, Lastilla M, Mengoni F et al.: Detection of interleukin-2 in addition to interferon-gamma discriminates active tuberculosis patients, latently infected individuals, and controls. Clin Microbiol Infect 2010, 16: 1282-1284.
26. Millington KA, Innes JA, Hackforth S, Hinks TS, Deeks JJ, Dosanjh DP et al.: Dynamic relationship between IFN-gamma and IL-2 profile of Mycobacterium tuberculosis-specific T cells and antigen load. J Immunol 2007, 178: 5217-5226.
27. Sargentini V, Mariotti S, Carrara S, Gagliardi MC, Teloni R, Goletti D et al.: Cytometric detection of antigen-specific IFN-gamma/IL-2 secreting cells in the diagnosis of tuberculosis. BMC Infect Dis 2009, 9: 99.
28. Sester U, Fousse M, Dirks J, Mack U, Prasse A, Singh M et al.: Whole-blood flow-cytometric analysis of antigen-specific CD4 T-cell cytokine profiles distinguishes active tuberculosis from non-active states. PLoS ONE 2011, 6: e17813.
29. Khutso G.Phalane, Magdalena Kriel, Andre G.Loxton, Angela Menezes, Kim Stanley, Gian D.van der Spuy et al.. Differential Expression of Host Biomarkers in Saliva and Serum Samples from Individuals with Suspected Pulmonary Tuberculosis. Mediators of Inflammation 2013, Article ID 981984. 2013. Ref Type: Journal (Full)
30. Leyten EM, Lin MY, Franken KL, Friggen AH, Prins C, van Meijgaarden KE et al.: Human T-cell responses to 25 novel antigens encoded by genes of the dormancy regulon of Mycobacterium tuberculosis. Microbes Infect 2006, 8: 2052-2060.
31. Corstjens PL, Chen Z, Zuiderwijk M, Bau HH, Abrams WR, Malamud D et al.: Rapid assay format for multiplex detection of humoral immune responses to infectious disease pathogens (HIV, HCV, and TB). Ann N Y Acad Sci 2007, 1098: 437-445.
32. Corstjens PL, Zuiderwijk M, Tanke HJ, van der Ploeg-van Schip JJ, Ottenhoff TH, Geluk A: A user-friendly, highly sensitive assay to detect the IFN-gamma secretion by T cells. Clin Biochem 2008, 41: 440-444.
33. Corstjens PL, van LL, Zuiderwijk M, Kornelis D, Tanke HJ, Deelder AM et al.: Up-converting phosphor technology-based lateral flow assay for detection of Schistosoma circulating anodic antigen in serum. J Clin Microbiol 2008, 46: 171-176.
34. Corstjens PL, de Dood CJ, van der Ploeg-van Schip JJ, Wiesmeijer KC, Riuttamaki T, van Meijgaarden KE et al.: Lateral flow assay for simultaneous detection of cellular- and humoral immune responses. Clin Biochem 2011, 44: 1241-1246.

The following numbered paragraphs define particular embodiments of the present disclosure:
1. A method for the diagnosis of tuberculosis, the method comprising the step of:
   testing an unstimulated blood sample from a subject suspected of having tuberculosis for at least 4 biomarkers, wherein the biomarkers are selected from the group consisting of c-reactive protein (CRP), pre-albumin, interferon (IFN)-γ, complement factor H, apolipoprotein (APO)-A1, interferon inducible protein (IP)-10, complement C3, alpha-2-macroglobulin, APO-CIII, APO-E, interferon (IFN)-α2, tumour necrosis factor (TNF)-α, epidermal growth factor (EGF), macrophage inflammatory protein (MIP)-1β, soluble CD40 ligand (sCD40L), transforming growth factor (TGF)-a, vascular endothelial growth factor (VEGF), interleukin 1 receptor antagonist (IL-1ra), matrix metallo-proteinase (MMP)-2, matrix metallo-proteinase (MMP)-9, haptoglobulin, serum amyloid A (SAA), procalcitonin (PCT), ferritin, tissue plasminogen activator (TPA), fibrinogen and serum amyloid A (SAA).
2. A method according to paragraph 1, wherein the biomarkers are selected from the group consisting of: CRP, prealbumin, IFN-γ, complement factor H, apolipoprotein A1, IP-10, SAA, ferritin, fibrinogen, TGF-α, TPA, VEGF, PCT and TNF-α.
3. A method according to either of paragraphs 1 or 2, which comprises testing the sample for at least 5 of said biomarkers.
4. A method according to any one of paragraphs 1 to 3, which comprises testing the sample for at least 6 of said biomarkers.
5. A method according to any one of paragraphs 1 to 4, wherein at least 2 of the biomarkers are selected from the group consisting of CRP, prealbumin, IFN-γ, complement factor H, apolipoprotein A1 and IP-10 and the other biomarkers are selected from the group consisting of SAA, Ferritin, Fibrinogen, TGF-α, TPA, VEGF, PCT and TNF-α.
6. A method according to any one of paragraphs 1 to 5, wherein at least 3 of the biomarkers are selected from the group consisting of CRP, prealbumin, IFN-γ, complement factor H, apolipoprotein A1 and IP-10 and the other biomarkers are selected from the group consisting of SAA, Ferritin, Fibrinogen, TGF-α, TPA, VEGF, PCT and TNF-α.
7. A method according to any one of paragraphs 3 to 6, wherein at least 4 of the biomarkers are selected from the group consisting of CRP, prealbumin, IFN-γ, complement factor H, apolipoprotein A1 and IP-10 and the other biomarkers are selected from the group consisting of SAA, Ferritin, Fibrinogen, TGF-α, TPA, VEGF, PCT and TNF-α.
8. A method according to any one of paragraphs 4 to 7, wherein at least 5 of the biomarkers are selected from the group consisting of CRP, prealbumin, IFN-γ, complement factor H, apolipoprotein A1 and IP-10 and the other biomarkers are selected from the group consisting of SAA, Ferritin, Fibrinogen, TGF-α, TPA, VEGF, PCT and TNF-α.
9. A method according to any one of paragraphs 1 to 8, wherein the biomarkers are selected from the group consisting of CRP, prealbumin, IFN-γ, complement factor H, apolipoprotein A1 and IP-10.
10. A method according to any one of paragraphs 1 to 9, which comprises testing the sample for the following 6 biomarkers: CRP, prealbumin, IFN-γ, complement factor H, apolipoprotein A1 and IP-10.
11. A method according to any one of paragraphs 1 to 10, wherein a capture agent is used to bind each of the biomarkers.
12. A method according to paragraph 11, wherein the capture agents are selected from the group consisting of antibodies, affybodies, ankyrin repeat proteins, armadillo repeat proteins, nucleic acid aptamers, modified nucleic acid aptamers, peptides, modified peptides, carbohydrate ligands, synthetic ligands, and synthetic polymers.
13. A method according to either of paragraphs 11 or 12, wherein one or more indicators are provided to indicate when binding of each of the capture agents and biomarkers occurs.
14. A method according to paragraph 13, wherein the indicator is selected from the group consisting of a calorimetric, electrochemical, chromogenic, optical, fluorescent and a radio-labeled indicator.
15. A method according to any one of paragraphs 1 to 14, wherein a measured signal which equates to a level of biomarker in the sample which is higher than a threshhold level of the same biomarker is an indicator of tuberculosis disease for the following biomarkers: IFN-α2, CRP, IFN-γ, IL-1ra, IP-10, TGF-α, TNF-α, VEGF, complement factor H, complement C3, PCT, SAA, ferritin, fibrinogen, TPA or MMP-9.
16. A method according to any one of paragraphs 1 to 14, wherein a measured signal which equates to a level of biomarker in the sample which is lower than a threshhold level of the same biomarker is an indicator of tuberculosis disease for the following biomarkers: APO-A1, APO-CIII, pre-albumin or MMP-2.
17. A method according to either of paragraphs 15 or 16, wherein the threshold level of the biomarkers corresponds to levels of the same biomarkers in subjects who do not have tuberculosis.
18. A method according to any one of paragraphs 1 to 17, wherein the sample is whole blood, blood plasma or serum.
19. A method according to any one of paragraphs 1 to 18, wherein the tuberculosis is pulmonary tuberculosis.
20. A device for diagnosing tuberculosis according to the method of any one of paragraphs 1 to 19, the device comprising:
   a means for receiving a blood sample from a subject;
   capture agents for binding at least 4 of the biomarkers of any one of paragraphs 1 to 10 if the biomarkers are present in the sample; and
   at least one indicator which indicates when the capture agents bind to the biomarkers.
21. A device according to paragraph 20, wherein the capture agents are selected from the group consisting of antibodies, affybodies, ankyrin repeat proteins, armadillo repeat proteins, nucleic acid aptamers, modified nucleic acid aptamers, peptides, modified peptides, carbohydrate ligands, synthetic ligands, and synthetic polymers.
22. A device according to either of paragraphs 20 or 21, wherein the indicator indicates binding of the capture agent to the biomarker by electrical, acoustic, optical or mechanical methods.
23. A device according to any one of paragraphs 20 to 22, which includes measuring means for measuring the levels of the detected biomarkers.
24. A device according to any one of paragraphs 20 to 23, which further includes amplifying means for increasing the sensitivity of the detection of the biomarkers.
25. A device according to any one of paragraphs 20 to 24, which is a hand-held point-of-care device.
26. A kit for diagnosing tuberculosis in a blood sample from a subject according to the method of any one of paragraphs 1 to 19, the kit comprising:
   a means of receiving a sample from a patient;
   at least one capture agent for binding at least 4 of the biomarkers of any of paragraphs 1 to 19; and
   at least one indicator which indicates when the capture agents bind to the biomarkers.
27. A kit according to paragraph 26, which further includes a device of any one of paragraphs 20 to 25.

## Claims

1. A method for the diagnosis of tuberculosis, the method comprising the step of:
testing an unstimulated blood sample from a subject suspected of having tuberculosis for at least 4 biomarkers, wherein the biomarkers are selected from the group consisting of complement factor H, c-reactive protein (CRP), pre-albumin, interferon (IFN)-γ, apolipoprotein (APO)-A1 and interferon inducible protein (IP)-10.

2. A method according to claim 1, which comprises testing the sample for at least 5 of said biomarkers.

3. A method according to claim 1, which comprises testing the sample for at least 4 biomarkers selected from the group consisting of CRP, prealbumin, IFN-γ, complement factor H, apolipoprotein A1 and IP-10 and at least one other biomarker selected from the group consisting of SAA, Ferritin, Fibrinogen, TGF-α, TPA, VEGF, PCT and TNF-α.

4. A method according to any one of claims 1 to 3, which comprises testing the sample for at least 5 biomarkers selected from the group consisting of CRP, prealbumin, IFN-γ, complement factor H, apolipoprotein A1 and IP-10 and at least one other biomarker selected from the group consisting of SAA, Ferritin, Fibrinogen, TGF-α, TPA, VEGF, PCT and TNF-α.

5. A method according to any one of claims 1 to 4, which comprises testing the sample for the following biomarkers: CRP, SAA, IFN-γ, complement factor H, apolipoprotein A1 and IP-10.

6. A method according to either of claims 1 or 2, which comprises testing the sample for the following 6 biomarkers: CRP, prealbumin, IFN-γ, complement factor H, apolipoprotein A1 and IP-10.

7. A method according to claim 6, which additionally comprises testing the sample for SAA or Ferritin.

8. A method according to any one of claims 1 to 7, wherein a capture agent is used to bind each of the biomarkers.

9. A method according to claim 8, wherein one or more indicators are provided to indicate when binding of each of the capture agents and biomarkers occurs.

10. A method according to any one of claims 1 to 9, wherein a measured signal which equates to a level of biomarker in the sample which is higher than a threshhold level of the same biomarker is an indicator of tuberculosis disease for the following biomarkers: CRP, IFN-γ, IP-10, TGF-α, TNF-α, VEGF, complement factor H, PCT, SAA, ferritin, fibrinogen and TPA.

11. A method according to any one of claims 1 to 9, wherein a measured signal which equates to a level of biomarker in the sample which is lower than a threshhold level of the same biomarker is an indicator of tuberculosis disease for the following biomarkers: APO-A1 and pre-albumin.

12. A method according to any one of claims 1 to 11, wherein the sample is whole blood, blood plasma or serum.

13. A method according to any one of claims 1 to 12, wherein the tuberculosis is pulmonary tuberculosis.

## Patentansprüche

1. Verfahren für die Diagnose von Tuberkulose, wobei das Verfahren die folgenden Schritte umfasst:
Testen einer nicht stimulierten Blutprobe von einer Versuchsperson, bei der der Verdacht besteht, dass sie Tuberkulose hat, auf mindestens 4 Biomarker, wobei die Biomarker aus der Gruppe ausgewählt werden, die besteht aus Komplementfaktor H, C-reaktivem Protein (CRP), Präalbumin, Interferon (IFN)-γ, Apolipoprotein-(APO)-A1 und interferoninduzierbarem Protein (IP)-10.

2. Verfahren nach Anspruch 1, das das Testen der Probe auf mindestens 5 der genannten Biomarker umfasst.

3. Verfahren nach Anspruch 1, das das Testen der Probe auf mindestens 4 Biomarker umfasst, die aus der Gruppe ausgewählt werden, welche aus CRP, Präalbumin, IFN-γ, Komplementfaktor H, Apolipoprotein A1 und IP-10 besteht, und auf mindestens einen anderen Biomarker, der aus der Gruppe ausgewählt wird, die aus SAA, Ferritin, Fibrinogen, TGF-α, TPA, VEGF, PCT und TNF-α besteht.

4. Verfahren nach einem der Ansprüche 1 bis 3, das das Testen der Probe auf mindestens 5 Biomarker umfasst, die aus der Gruppe ausgewählt werden, die aus CRP, Präalbumin, IFN-y, Komplementfaktor H, Apolipoprotein A1 und IP-10 besteht, und mindestens einen anderen Biomarker, der aus der Gruppe ausgewählt wird, die aus SAA, Ferritin, Fibrinogen, TGF-α, TPA, VEGF, PCT und TNF-α besteht.

5. Verfahren nach einem der Ansprüche 1 bis 4, das das Testen der Probe auf die folgenden Biomarker umfasst: CRP, SAA, IFN-y, Komplementfaktor H, Apolipoprotein A1 und IP-10.

6. Verfahren nach Anspruch 1 oder 2, das das Testen der Probe auf die folgenden 6 Biomarker umfasst: CRP, Präalbumin, IFN-γ, Komplementfaktor H, Apolipoprotein A1 und IP-10.

7. Verfahren nach Anspruch 6, das zusätzlich das Testen der Probe auf SAA oder Ferritin umfasst.

8. Verfahren nach einem der Ansprüche 1 bis 7, wobei ein Einfangmittel verwendet wird, um jeden der Biomarker zu binden.

9. Verfahren nach Anspruch 8, wobei einer oder mehrere Indikatoren zum Anzeigen bereitgestellt werden, wenn das Binden jedes der Einfangmittel und Biomarker eintritt.

10. Verfahren nach einem der Ansprüche 1 bis 9, wobei ein Messsignal, welches einem Biomarker-Pegel in der Probe entspricht, der größer als ein Schwellwertpegel desselben Biomarkers ist, ein Indikator für Tuberkulose für die folgenden Biomarker ist: CRP, IFN-y, IP-10, TGF-α, TNF-α, VEGF, Komplementfaktor H, PCT, SAA, Ferritin, Fibrinogen und TPA.

11. Verfahren nach einem der Ansprüche 1 bis 9, wobei ein Messsignal, welches einem Biomarker-Pegel in der Probe entspricht, der größer als ein Schwellwertpegel desselben Biomarkers ist, ein Indikator für Tuberkulose für die folgenden Biomarker ist: APO-A1 und Präalbumin.

12. Verfahren nach einem der Ansprüche 1 bis 11, wobei die Probe Vollblut, Blutplasma oder Serum ist.

13. Verfahren nach einem der Ansprüche 1 bis 12, wobei die Tuberkulose Lungentuberkulose ist.

## Revendications

1. Un procédé pour le diagnostic de la tuberculose, le procédé comprenant l'étape consistant à :
tester un échantillon de sang non stimulé d'un sujet suspecté d'avoir la tuberculose pour au moins 4 biomarqueurs, dans lequel les biomarqueurs sont choisis dans le groupe constitué par le facteur H du complément, la protéine C réactive (CRP), la préalbumine, l'interféron (IFN)-γ, l'apolipoprotéine (APO)-A1 et la protéine inductible par interféron (IP)-10.

2. Un procédé selon la revendication 1, qui comprend le test de l'échantillon pour au moins 5 desdits biomarqueurs.

3. Un procédé selon la revendication 1, qui comprend le test de l'échantillon pour au moins 4 biomarqueurs choisis dans le groupe constitué par la CRP, la préalbumine, l'IFN-γ, le facteur H du complément, l'apolipoprotéine A1 et l'IP-10 et au moins un autre biomarqueur choisi parmi le groupe constitué par le SAA, la ferritine, le fibrinogène, le TGF-α, le TPA, le VEGF, la PCT et le TNF-α.

4. Un procédé selon l'une quelconque des revendications 1 à 3, qui comprend le test de l'échantillon pour au moins 5 biomarqueurs choisis dans le groupe constitué par la CRP, la préalbumine, I'IFN-γ, le facteur H du complément, l'apolipoprotéine A1 et l'IP-10 et au moins un autre biomarqueur choisi parmi le groupe constitué par le SAA, la ferritine, le fibrinogène, le TGF-α, le TPA, le VEGF, la PCT et le TNF-α.

5. Un procédé selon l'une quelconque des revendications 1 à 4, qui comprend le test de l'échantillon pour les biomarqueurs suivants : CRP, SAA, IFN-γ, facteur H du complément, apolipoprotéine A1 et IP-10.

6. Un procédé selon l'une ou l'autre des revendications 1 ou 2, qui comprend le test de l'échantillon pour les 6 biomarqueurs suivants : CRP, préalbumine, IFN-γ, facteur H du complément, apolipoprotéine A1 et IP-10.

7. Un procédé selon la revendication 6, qui comprend en outre le test de l'échantillon pour le SAA ou la ferritine.

8. Un procédé selon l'une quelconque des revendications 1 à 7, dans lequel un agent de capture est utilisé pour lier chacun des biomarqueurs.

9. Un procédé selon la revendication 8, dans lequel un ou plusieurs indicateurs sont fournis pour indiquer quand la liaison de chacun des agents de capture et biomarqueurs se produit.

10. Un procédé selon l'une quelconque des revendications 1 à 9, dans lequel un signal mesuré qui équivaut à un niveau de biomarqueur dans l'échantillon qui est supérieur à un niveau seuil du même biomarqueur est un indicateur de tuberculose pour les biomarqueurs suivants : CRP, IFN-γ, IP-10, TGF-α, TNF-α, VEGF, facteur H du complément, PCT, SAA, ferritine, fibrinogène et TPA.

11. Un procédé selon l'une quelconque des revendications 1 à 9, dans lequel un signal mesuré qui équivaut à un niveau de biomarqueur dans l'échantillon qui est inférieur à un niveau seuil du même biomarqueur est un indicateur de tuberculose pour les biomarqueurs suivants : APO-A1 et préalbumine.

12. Un procédé selon l'une quelconque des revendications 1 à 11, dans lequel l'échantillon est du sang total, du plasma sanguin ou du sérum.

13. Un procédé selon l'une quelconque des revendications 1 à 12, dans lequel la tuberculose est une tuberculose pulmonaire.
